# EUROPÄISCHE PATENTANMELDUNG

(11) **EP 2 103 308 A1**
(43) Veröffentlichungstag der Anmeldung: **23.09.2009**
(21) Anmeldenummer: 08005273.1
(22) Anmeldetag: 20.03.2008
(51) Int. Cl.: A61K 35/76, A61P 31/04

(54) **Verfahren zur Herstellung eines Gemisches von Bakteriophagen und deren Verwendung für die Therapie von Antibiotika-resistenten Staphylococcen**

(71) Anmelder: PhytoLine GmbH, 41749 Viersen (DE)
(72) Erfinder: Müller, Robert, 53332 Bornheim (DE)
(74) Vertreter: Keller, Günter

(57) **Zusammenfassung**

Offenbart wird ein Verfahren zur Herstellung eines Gemisches von Bakteriophagen, bei dem wenigstens zwei unterschiedliche Bakteriophagenstämme separat vermehrt wurden, wobei ein Staphylococcus-Stamm mit je einem Stamm eines Bakteriophagen, der Staphylococcen lysieren kann, in einer Vorkultur angezogen und dann in einer Hauptkultur weiter vermehrt und anschließend gereinigt wird und die verschiedenen Stämme anschließend gemischt werden, wobei das eingesetzte Gemisch von Bakteriophagen wenigstens zwei verschiedene Serotypen von Bakteriophagen enthält, die spezifisch Bakterien der Spezies Staphylococcus lysieren.

## Beschreibung

Bakterielle Infektionen verursachen viele, zum Teil tödlich verlaufende Erkrankungen. Durch die Entwicklung von Antibiotika konnten viele dieser bakteriellen Infektionen erfolgreich bekämpft werden. Der umfassende Einsatz von verschiedenen Antibiotika führte aber zu dem Auftreten von Bakterien, die häufig nicht nur gegen ein Antibiotikum, sondern gegen eine Vielzahl von Antibiotika resistent sind. Derartige hochresistente Bakterienstämme führen immer mehr zu schwerwiegenden Problemen, gerade in Krankenhäusern, weil dort auch durch die ärztlichen Behandlungen Patienten mit derartigen Stämmen infiziert werden können. Wenn diese Stämme gegen die meisten, üblicherweise eingesetzten Antibiotika resistent sind, kann dies zu äußerst schwerwiegenden Erkrankungen führen, die nicht selten tödlich enden.

Im Rahmen der vorliegenden Erfindung wird auf eine alternative Bekämpfung von Bakterien zurückgegriffen. So wie es Viren gibt, die Pflanzen und Tiere befallen und dabei nicht selten äußerst schwere Erkrankungen hervorrufen, gibt es auch Viren, die speziell Bakterien befallen. Diese Viren werden Bakteriophagen genannt. Sie sind üblicherweise spezifisch für bestimmte Bakterienarten und binden häufig an diejenigen Bereiche der Bakterienzellwand, an denen üblicherweise die Aufnahme von Nährstoffen (beispielsweise Zucker u.ä.) erfolgt.

Nach der Adsorption der Bakteriophagen an die Bakterienzellwand injizieren die Bakteriophagen ihre Nucleinsäure in das Bakterium. Dort vermehrt sich die Nucleinsäure und es werden Bakteriophagen-spezifische Proteine gebildet. Wenn genug Kopien der Bakteriophagen-Nucleinsäure und ausreichend Bakteriophagen-Protein in der Bakterienzelle vorhanden ist, lysiert diese die Bakterienzelle und die gebildeten Bakteriophagen werden in großer Zahl in die Umgebung freigesetzt. Dann kann der Infektionszyklus von neuem beginnen.

Die Verwendung von Bakteriophagen zur Therapie von bestimmten Erkrankungen ist schon seit mehreren Jahrzehnten bekannt. Insbesondere in osteuropäischen Ländern wurde diese Art der Bekämpfung von Bakterien entwickelt. In der WO 2004/052274 wird ein Verfahren zur Herstellung von Bakteriophagen-Zusammensetzungen beschrieben, die bei der Therapie von bakteriellen Infektionen, insbesondere solchen, die durch Pseudomonas aerogenosa hervorgerufen wurden, eingesetzt werden können. Die Anzucht der Bakteriophagen wird dort in einem halbfesten Kulturmedium durchgeführt.

Die WO 97/39111 beschreibt ein Verfahren zur Herstellung von Bakteriophagen mit einem breiten Wirtsspektrum und deren Einsatz in der Therapie.

In der WO 01/50866 wird der Einsatz eines Bakteriophagen-Cocktails zur Vernichtung von Bakterien auf verschiedenen festen Oberflächen, wie beispielsweise Operationsräumen oder auch Anlagen zur Tierhaltung, sowie Schlachthöfen offenbart.

Gegenstand der vorliegenden Erfindung ist ein Verfahren zur Herstellung eines Gemisches von Bakteriophagen, bei dem zunächst ein Staphylococcus-Stamm mit einem Bakteriophagen, der Staphylococcen lysieren kann, in einer Vorkultur angezogen und dann in einer Hauptkultur weiter vermehrt werden. Mehrere derartige Bakteriophagen werden dann zu einem Gemisch vereinigt.

Das eingesetzte Gemisch von Bakteriophagen enthält wenigstens zwei, bevorzugt wenigstens drei verschiedene Serotypen von Bakteriophagen, die spezifisch Bakterien der Spezies Staphylococcus lysieren. Bevorzugt eingesetzte Bakteriophagenstämme stammen aus der Phagenklassen Myoviridae und Podoviridae.

Es ist ein wesentlicher Aspekt der vorliegenden Erfindung, dass mehrere verschiedene Bakteriophagen eingesetzt werden, die alle die gemeinsame Eigenschaft haben, dass sie Staphylococcen lysieren können. Insbesondere müssen die eingesetzten Bakteriophagen solche Staphylococcen lysieren, die an human- und/oder tierpathogenen Erkrankungen beteiligt sind. Besonders bevorzugt lysieren die erfindungsgemäß eingesetzten Bakteriophagen solche Staphylococcen, insbesondere Staphylococcus aureus-Stämme, die Antibiotika-, insbesondere Methicillin-resistent sind. Es ist ein wesentlicher Aspekt der vorliegenden Erfindung, dass in dem Bakteriophagen-Gemisch mehrere verschiedene Bakteriophagen vorhanden sind. Es kann sich hierbei um solche Bakteriophagen handeln, die spezifisch Staphylococcus aureus-Stämme lysieren und es können auch solche Bakteriophagen eingesetzt werden, die auch andere Bakterienarten lysieren, wobei aber derartige Stämme bevorzugt nur zusätzlich zu einem Gemisch von Bakteriophagen eingesetzt werden, die wenigstens zwei unterschiedlichen Serotypen angehören.

Die bei dem erfindungsgemäß eingesetzten Verfahren verwendeten Bakteriophagen können entweder genau charakterisierte Bakteriophagen sein oder sie können aus einem Wildisolat herstammen. Hierzu werden nach üblichen Verfahren der Bakteriophagen-Isolierung ausgehend von einer geeigneten Quelle die Bakteriophagen durch gemeinsame Anzucht mit Staphylococcenstämmen zunächst angereichert und dann in dem erfindungsgemäßen Verfahren eingesetzt.

Bei dem erfindungsgemäßen Verfahren wird zur Vermehrung der Bakteriophagen aus Sicherheitsgründen bevorzugt ein Methicillin-sensitiver Staphylococcus aureus-Stamm eingesetzt.

Gerade bei problematischen Infektionen in Krankenhäusern werden häufig Methicillinresistente Staphylococcus aureus-Stämme angetroffen, die häufig als MRSA abgekürzt werden. Erfindungsgemäß wird zur Vermehrung der Bakteriophagen-Mischung aber bevorzugt ein Methicillin-sensitiver Staphylococcus aureus (MSSA)-Stamm eingesetzt. Der bevorzugte Stamm wurde zwischenzeitlich bei der Deutschen Sammlung für Mikroorganismen nach dem Budapester Vertrag unter der Hinterlegungsnummer DSM 18421 hinterlegt.

Ein besonders wichtiger Vorteil des erfindungsgemäß eingesetzten Staphylococcus aureus-Stammes zur Vermehrung der Bakteriophagen ist, dass in diesem Stamm insbesondere folgende Virulenzfaktoren nicht vorhanden sind. Es handelt sich um folgende Toxin-Determinanten, die auf übertragbaren Elementen lokalisiert sind:

| | |
|---|---|
| tst: | 161/93 |
| sea: | 619/93 |
| seb: | 62/92 |
| sec: | 1229/93 |
| sed: | 1634/93 |
| see: | FRJ918 |
| eta: | 1437/00 |
| eta, etb: | 1004/00 |
| lukS-lukF: | 3925/02 |
| cna: | 2773/03 |
| seg: | N315 |
| seg und seh: | 2773/03. |

Dadurch können auch von dem Vermehrungsstamm keine Virulenzfaktoren auf die Bakteriophagen übertragen werden und die Bakteriophagen-Vermehrung ist in dieser Hinsicht besonders sicher.

Ein anderer Vorteil des erfindungsgemäß eingesetzten Vermehrungsstammes ist, dass sich die Eigenschaften der in dem Stamm vermehrten Bakteriophagen auch nach mehreren Passagen nicht verändern.

Für die erfindungsgemäße Herstellung des Bakteriophagen-Gemisches wird in dem ersten Schritt eine Vorkultur des eingesetzten Staphylococcus aureus-Stamms angezogen. Wenn die Bakterien eine geeignete optische Dichte erreicht haben, wird der zu vermehrende Bakteriophage zugegeben, wobei in der Vorkultur ein Verhältnis von Bakterien zu Bakteriophagen von etwa 10⁵ bis 10² Bakterien pro 1 Phage eingestellt wird.

Die Vorkultur wird zusammen mit den Bakteriophagen solange angezogen, bis die Lyse der Bakterien beginnt. Dann wird die Vorkultur, die die Bakteriophagen enthält, zu einer Hauptkultur von Staphylococcen gegeben, wobei bevorzugt ein solches Verhältnis eingestellt wird, dass die Bakterien im deutlichen Überschuss vorhanden sind. Besonders bevorzugt wird ein Bereich von etwa 50 bis 1000 Bakterien pro Bakteriophage. Durch diesen Überschuss wird erreicht, dass sämtliche Bakteriophagen, die sich in der Vorkultur befinden, ein geeignetes Bakterium finden, in dem sie sich vermehren können.

Das Verhältnis von Bakteriophagen zu Bakterien wird auch als sogenannter MOI-Wert (multiplicity of infection) bezeichnet. In einer bevorzugten Ausführungsform liegt der MOI-Wert bei der Vorkultur sowie der Hauptkultur zwischen 10⁻⁶ und 1.

Üblicherweise wird bei dem erfindungsgemäßen Verfahren die Hauptkultur in einem Fermenter geeigneter Größe durchgeführt. Bei den Fermentern können die einzelnen Umgebungsparameter wie Luftzufuhr, pH-Wert, Nährstoffzugabe u.ä. kontinuierlich gesteuert werden. In einer bevorzugten Ausführungsform erfolgt die Anzucht der Hauptkultur unter reduzierter Luftzufuhr und weiterhin wird der pH-Wert bevorzugt in einem Bereich zwischen 5,8 und 7,8 gehalten.

Nach der Fermentation werden die Bakteriophagen aus der Fermentationsbrühe üblicherweise dadurch erhalten, dass zunächst das Fermentationsmedium abzentrifugiert und/oder abfiltriert wird, so dass von den lysierten Bakterien herstammende Zellbestandteile abgetrennt werden. Der Überstand wird dabei bevorzugt durch ein oder mehrere Filter filtriert, wobei die Porengröße so gewählt ist, dass bei der ersten Filtration noch verbleibende Bakterien die Filter nicht passieren können. Die Porengröße dieser Filter liegt bevorzugt in einem Bereich zwischen 0,1 und 0.45 µm und bevorzugt zwischen 0,1 und 0,2 µm. Die Bakteriophagen können diese Filtergröße aufgrund ihrer kleineren Größe passieren. Es muss allerdings darauf geachtet werden, dass solche Filter gewählt werden, an denen die Bakteriophagen möglichst nicht adsorbieren, da dies anderenfalls zu Filterverstopfungen und zu einer Verschlechterung der Ausbeute an Phagen führen könnte.

In einem weiteren Schritt wird bevorzugt die Phagen enthaltende Lösung so diafiltriert, dass die Bakteriophagen die Poren nicht passieren können und dadurch in der Lösung angereichert werden. Durch übliche mikrobiologische Bestimmungsmethoden kann die Konzentration der Bakteriophagen ohne weiteres ermittelt werden. Bevorzugt enthält die Lösung eine Konzentration von wenigstens 10¹⁰ PFU (plaque-forming units) pro ml.

Im Rahmen der vorliegenden Erfindung wird ein Gemisch von wenigstens zwei oder mehr Bakteriophagen eingesetzt. Ein derartiges Gemisch wird bevorzugt so hergestellt, dass zunächst von jedem einzusetzenden Phagen eine Lösung mit einer definierten Phagenkonzentration hergestellt wird und diese Lösungen werden dann im gewünschten Verhältnis miteinander gemischt. Diese Bakteriophagen können denselben Phagenklassen oder auch unterschiedlichen Phagenklassen zugeordnet werden. In einer besonders bevorzugten Ausführungsform besteht das Bakteriophagen-Gemisch aus zwei unterschiedlichen Klassen der Myoviridae. Besonders bevorzugt weist das Bakteriophagen-Gemisch einerseits den Bakteriophagen vom Typ KP3 und/oder den Bakteriophagen mit der Bezeichnung MP13.3 auf. Daneben kann das Gemisch auch noch weitere geeignete Bakteriophagen umfassen.

Die beiden bevorzugten Bakteriophagen wurden am 18. Oktober 2006 bei der deutschen Sammlung von Mikroorganismen und Zellkulturen GmbH, Braunschweig, hinterlegt. Dem Bakteriophagenstamm MP13.3 wurde die Hinterlegungsnummer DSM 18722 und dem Bakteriophagenstamm mit der Bezeichnung KP3 die Hinterlegungsnummer DSM 18723 zugeteilt. Die Hinterlegung erfolgte nach Budapester Vertrag.

Das Bakteriophagen-Gemisch wird derart hergestellt, dass zunächst jeder Bakteriophagenstamm einzeln in den Wirtsbakterien vermehrt wird. Nach Reinigung und Titerbestimmung der jeweiligen Bakteriophagen-Präparation wird dann die Mischung hergestellt, wobei das Verhältnis zwischen den einzelnen Bakteriophagen im Bereich zwischen 1:10 bis 10:1 variieren kann. Bevorzugt wird eine Mischung eingesetzt, die in etwa zu gleichen Teilen aus den beiden Bakteriophagen besteht, wobei sich der Anteil jeweils auf die Plaque-bildenden Einheiten bezieht. Zu dieser Mischung können auch noch weitere Bakteriophagenstämme hinzugegeben werden, die separat vermehrt wurden.

Das erfindungsgemäße Phagengemisch kann für die Anwendung als Arzneimittel entsprechend weiterbehandelt werden. Bei diesen Schritten ist darauf zu achten, dass die Bakteriophagen nicht inaktiviert werden. Bevorzugt zugegeben werden Schutzsubstanzen wie beispielsweise verdickende Stoffe, beispielsweise Glycerin oder Polyethylenglykol. Derartige Zusatzstoffe sind dann bevorzugt, wenn Arzneimittel in Form von Flüssigkeiten verabreicht werden.

In einer bevorzugten Ausführungsform wird das erfindungsgemäße Phagengemisch als Arzneimittel so eingesetzt, dass der Bakteriophagenlösung noch wenigstens ein Enzym zugefügt wird, das eine synergistische Wirkung auf die Effizienz der Bakteriophagenlösung ausübt. Hierbei kann es sich um Enzyme handeln, die selbst bakterizid sind. Es können also Lysine, wie beispielsweise Lysozym eingesetzt werden, die aus verschiedenen Quellen, also aus Bakteriophagen, aus Bakterien (Endolysine, Exolysine) oder auch anderen Quellen stammen. Alternativ hierzu kann Lysozym eingesetzt werden, das beispielsweise aus Hühnereiern isoliert werden kann und die bakterielle Zellwand, insbesondere von Grampositiven Bakterien, lysieren kann. Andere Enzyme, die ebenfalls vorteilhafterweise eingesetzt werden können, gehören zu der Gruppe der Proteasen und/oder Peptidasen. Ein hierbei zu nennendes Beispiel wäre Papain. Schließlich können die Enzyme auch Nukleasen oder Glucosaminidasen sein, die zwar selbst nicht unmittelbar bakterizid wirken, aber die Bakteriophagenwirkung unterstützen.

In besonders bevorzugter Ausführungsform handelt es sich bei dem Enzym um die Protease Serralysin, die aus dem Stamm *Serratia marcescens* hergestellt wird. Ein anderes bevorzugtes Enzym ist die Nuklease Benzonase oder die Dornase α. In Beispiel 7 ist die überlegene Eigenschaft dieser Kombination mit einer biologischen Matrix (Speichel) offenbart.

Wenn das erfindungsgemäße Bakteriophagen-Gemisch in anderer Form appliziert werden soll, kann es auch vorteilhaft sein, das Gemisch zu lyophilisieren. Auch hier können geeignete Schutzkomponenten wie verschiedene Zucker zugegeben werden.

Die erfindungsgemäßen Arzneimittel können in Form einer Lösung, Tinktur, Salbe oder Lotion aufgebracht werden. In einer weiteren bevorzugten Ausführungsform enthält ein derartiges Arzneimittel neben dem Phagengemisch auch ein Antibiotikum, das vorzugsweise gegen Gram-positive Bakterien wirksam ist. Hintergrund dieser Kombination ist, dass die Methicillin-resistenten Bakterien durch das erfindungsgemäße Phagengemisch lysiert werden sollen. Gleichzeitig soll aber verhindert werden, dass eventuell andere Bakterien, die nicht gegen Antibiotika resistent sind, wieder hochwachsen können. Da häufig Antibiotika-Resistenzen bei Staphylococcen auf Plasmiden kodiert sind, könnten an sich Antibiotikasensitive Bakterien die Plasmide aus den lysierten Bakterien aufnehmen. Dies wird durch die bevorzugte Kombination verhindert.

Das erfindungsgemäße Phagengemisch wird bevorzugt zur Herstellung eines Arzneimittels verwendet, das zur Therapie von solchen Infektionen dient, die durch Antibiotika-resistente Staphylococcus aureus-Stämme verursacht werden. In einer bevorzugten Ausführungsform handelt es sich bei derartigen Infektionen um solche, die auf der Haut und/oder den Schleimhäuten auftreten. Diese Infektionen können durch die erfindungsgemäßen Bakteriophagen-Gemische leicht und wirksam therapiert werden. Ein Vorteil hierbei ist, dass das Bakteriophagen-Gemisch nicht intravenös und/oder intramuskulär verabreicht werden muss, sondern einfach auf die Wunde in Form einer Lösung, eines Pulvers oder Sprays bzw. einer Salbe aufgetragen werden kann.

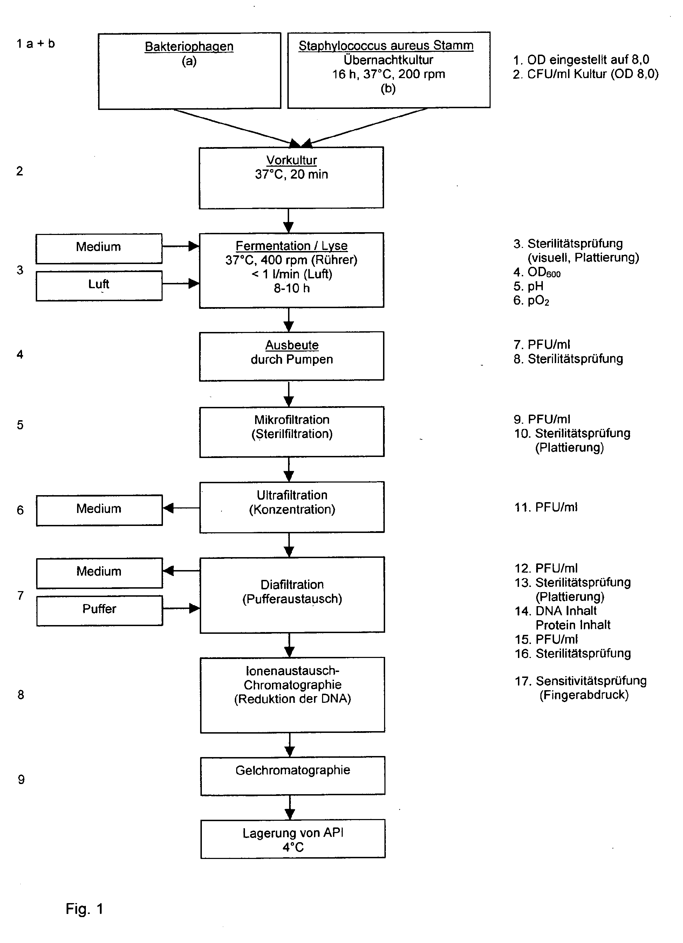

Das erfindungsgemäße Verfahren wird schematisch in Figur 1 dargestellt.

In einer bevorzugten Ausführungsform des erfindungsgemäßen Verfahrens wird zu der Bakteriophagen enthaltenden Lösung Benzonase zugegeben. Durch die Benzonase wird hochmolekulare DNA, die hauptsächlich von den lysierten Staphylococcen stammt, so gespalten, dass eine Infektiosität oder mögliche Rekombination mit Bruchstücken der Staphylococcen-DNA nicht zu befürchten ist. Andererseits hat die Benzonase einen positiven Einfluss auf die Bakteriophagenlösung im Hinblick auf die Viskosität. Daher wird die Benzonase bevorzugt entweder vor der Mikrofiltration oder nach der Diafiltration zugegeben. Wenn Benzonase zugegeben wird, empfiehlt es sich, nach der Reinigung mit der lonenaustausch-Chromatographie noch eine Gelchromatographie durchzuführen. Dadurch werden die noch verbliebenen Reste der Benzonase abgetrennt. Das Molekulargewicht der Benzonase liegt zwischen 30.000 und 40.000 Dalton. Es ist daher empfehlenswert, dass das für die Gelchromatographie eingesetzte Säulenmaterial ein Ausschlussvolumen von etwa 100.000 Dalton, bevorzugt etwa 50.000 Dalton aufweist.

Die nachfolgenden Beispiele sollen die vorliegende Erfindung erläutern.

### Beispiel 1: Phagenproduktion im Fermenter

### 1.1 Mikroorganismen

- Bakterien für Fermentation: MRSA M.03.02.0028, Gefrierkultur eines Stammes, der von Prof. Daschner, Freiburg, erhalten wurde. Der Stamm wurde bei der DSM unter der Nr. 18421 nach dem Budapester Vertrag hinterlegt.
- Bakterien für Plaquetest: Staphylococcus aureus M.03.02.0008 erhalten von Prof. Daschner, Freiburg. Der Stamm wurde bei der DSM nach dem Budapester Vertrag hinterlegt unter der Nr. DSM 18421.
- Phagen: Mutterphagen I, steril filtriert Konzentration: 1x10¹⁰ PFU/ml Es wurden jeweils separat die beiden Bakteriophagenstämme KP3 (DSM 18723) und MP13.3 (DSM 18722) angezogen.

### 1.2 Nährmedien

a) APS-LB Broth
   20 g Fertigmedium (Becton/Dickinson, Chargennr. 430510) werden eingewogen, in entionisiertem Wasser gelöst und auf 1 I Endvolumen aufgefüllt. Der natürliche pH-Wert des so hergestellten Mediums liegt bei ca. 6,8 und muss nicht eingestellt werden. Das Medium wird bei 121°C für 20 min sterilisiert und anschließend bei Raumtemperatur (RT; 20-22°C) aufbewahrt.
b) APS-LB Broth (Fermentation)
   20 g Fertigmedium (s.o.) + 2,5 g MgSO₄ (Roth, p.a.) werden eingewogen, in entionisiertem Wasser gelöst und auf 1 I Endvolumen aufgefüllt. Das Medium wird bei 121°C für 20 min in der Mediaprep sterilisiert und anschließend direkt in das sterilisierte Kulturgefäß eingefüllt.
c) LB -Medium
   10 g Trypton aus Casein (Roth, für die Mikrobiologie), 5 g NaCl (Roth, p.a.) und Hefeextrakt (Roth, für die Bakteriologie) werden in entionisiertem Wasser gelöst und auf 1 I Endvolumen aufgefüllt.
   Zur Herstellung von festem Nährmedium werden 15 g Agar Agar/l (Roth, hochrein) zugesetzt. Das Medium wird bei 121°C für 20 min sterilisiert. Die Agarplatten werden bei RT gelagert. Für den Versuch wurden Platten verwendet, die maximal 1 Woche unter diesen Bedingungen gelagert wurden.
d) Vogel-Johnson-Agar
   58 g Fertigmedium (Merck) wurden in 1 I warmem, entionisierten Wasser gelöst, anschließend bei 121°C für 20 min sterilisiert. Das Medium wurde auf ca. 50°C abgekühlt, dann wurden 0,24 g/l Kaliumtellurit (sterilfiltrierte Lösung) zugegeben. Das Medium wird in Petrischalen gefüllt, 30 min unter der Cleanbench getrocknet und anschließend bei 4°C verwahrt.
e) LB-Topagar
   1,0 g Trypton aus Casein
   0,5 g NaCl
   0,8 g Agar Agar
   werden in entionisiertem Wasser gelöst, auf 100 ml aufgefüllt und anschließend bei 121°C für 20 min sterilisiert. Die Aufbewahrung bis zur Verwendung erfolgt im Trockenschrank bei 60°C.

### 1.3 Puffer

a) 0,85 % NaCl
   0,85 g NaCl wurden in entionisiertem Wasser gelöst und auf 100 ml Endvolumen aufgefüllt. Der Puffer wird bei 121°C für 20 min sterilisiert.
b) Phagenpuffer
   20 mM Tris (pH 7,4), (Roth, Ultra Qualität)
   100 mM NaCl
   10 mM MgSO₄x7H₂O
      Die Herstellung erfolgte aus sterilen Stammlösungen und sterilem, entionisiertem Wasser. Die Lagerung erfolgte bei RT.
c) 1 M NaOH
   40,01 g NaOH (Roth) wurden in entionisiertem Wasser gelöst und auf 1 I Endvolumen aufgefüllt. Die Lösung wurde bei 121°C für 20 min sterilisiert, anschließend bei RT verwahrt.
d) pH-Eichlösungen
   pH 4,01 (Mettler Toledo)
   pH 7,00 (Mettler Toledo)
e) Sauerstoff-Sonde (pO₂-Sonde; misst den Sauerstoffpartialdruck eines Mediums) Zur Kalibrierung der pO₂-Sonde wird in der Taskleiste erst der Calibration-Button ausgewählt, dann im nächsten Fenster der pO₂-Button. Hier wird ebenfalls der Temperaturwert auf Raumtemperatur eingestellt bzw. die Temperatur der Lösungen eingegeben.
f) OD-Sonde
   OD-Sonde wird nicht geeicht, sondern nur der Nullpunkt bei jedem Lauf neu gesetzt für das jeweilige Medium. Da die Sonde bislang nicht linearisiert wurde, ist der externe Vergleich mit der OD₆₀₀ gemessen am Photometer unerlässlich.
g) pH-Messung
   Das pH-Meter wird vor der Messung angeschaltet und die benötigte Temperatur eingestellt. Das pH-Meter wird auf seinen Kalibrierungszustand untersucht und - falls notwendig - nach Herstellervorschrift kalibriert. Die Sonde wird aus der 3 M KCI-Lösung genommen und mit entionisiertem Wasser abgespült. Die Sonde wird mit einem Papiertuch abgetrocknet und in die zu messende Lösung gehalten. Der Messvorgang kann durch Schwenken oder Rühren der Lösung beschleunigt werden. Der gemessene Wert wird notiert, die Sonde aus der Lösung genommen und gründlich mit 70 % Ethanol, danach mit entionisiertem Wasser gespült, mit einem Papiertuch getrocknet und dann wieder in die 3 M KCI-Lösung zur Aufbewahrung gegeben.

### 1.5 Anzucht Bakterienstamm

- von dem unter 1.1 genannten Stamm wurde eine von für die Fermentationen angelegte Glycerin-Gefrierkultur aus dem -80°C Gefrierschrank entnommen und unter der Cleanbench angetaut
- 2 Impfösen Zellmaterial wurden in 20 ml APS LB-Medium angeimpft und bei 37°C und 200 rpm über Nacht schüttelnd bebrütet.

### 1.6 Fermentation

- die Kultur wurde vom 37°C Schüttelinkubator genommen
- 0,1 ml wurden zur OD-Messung entnommen, 1+9 mit Medium verdünnt (0,9 ml APS-LB + 0,1 ml Kultur) und ein OD₆₀₀ Mittelwert von 1,363 bestimmt
- über den Entnahmeschlauch wurde eine Probe des Mediums entnommen und in ein Eppi (= Reaktionsgefäß der Firma Eppendorf) gefüllt. Davon wurden jeweils 500 µl auf LB-Agar und Vogel-Johnson-Agar ausplattiert und bei 37°C über Nacht inkubiert.

### 1.7 Angleichung der Übernachtkultur auf OD 8,0

- die Kultur wurde mit Medium in einem sterilen Falcon-Röhrchen gemischt und es wurde durch Verdünnen mit sterilem Medium eine Optische Dichte von ca. 8,0 eingestellt.

### 1.8 Inkubation Bakterien/Phagen

- die einzusetzenden Phagen (1.1) wurden auf 2x10⁻² in Phagenpuffer in Eppis verdünnt (finales Volumen: 2 ml der 2x10⁻²-Verdünnung)
- als die Temperatur im Gefäß stabil war, wurden 15 ml eingestellte Bakterienkultur und 2 ml Phagen im Falcon-Röhrchen gemischt und 20 min bei 37°C inkubiert
- anschließend wurde die Bakterien/Phagenmischung über eine Kanüle in eine 20 ml Spritze gesogen und über das Septum im Deckel des Kulturgefäßes injiziert
- nach 3 min Rühren wurden die ersten Messwerte der Fermentation bestimmt.

### 1.9 CFU/mL-Bestimmung

- es wurde eine dekadische Verdünnungsreihe der auf OD 8,0 eingestellten Kultur nach folgendem Schema pipettiert:

**Tabelle 1**

| **10⁻¹** | **10⁻²** | **10⁻³** | **10⁻⁴** | **10⁻⁵** | **10⁻⁶** | **10⁻⁷** | **10⁻⁸** |
|---|---|---|---|---|---|---|---|
| 450 µl | 450 µl | 450 µl | 450 µl | 450 µl | 450 µl | 450 µl | 450 µl |
| NaCl | NaCl | NaCl | NaCl | NaCl | NaCl | NaCl | NaCl |
| 0,85 % | 0,85 % | 0,85 % | 0,85 % | 0,85 % | 0,85 % | 0,85 % | 0,85 % |
| + | + | + | + | + | + | + | + |
| 50 µl Kultur | 50 µl 10⁻¹ | 50 µl 10⁻² | 50 µl 10⁻³ | 50 µl 10⁻⁴ | 50 µl 10⁻⁵ | 50 µl 10⁻⁶ | 50 µl 10⁻⁷ |

- 2x100 µl der Verdünnungsstufen 10⁻⁶-10⁻⁸ wurden auf LB-Agar ausplattiert (Drigalski-Ausstrich)
- die Platten wurden ü.N. bei 37°C am nächsten Tag inkubiert

### 1.10 Fermentation

- nach Fermentationsstart wurden alle 30-60 min die Parameter abgelesen bzw. gemessen.
- die OD₆₀₀-Messungen erfolgten gegen das Fermentationsmedium als Blindwert
- die Probenahmen (jeweils 8 ml) während der Fermentation erfolgten über den Entnahmeschlauch jeweils mittels einer sterilen 10 ml Spritze
- von den 8 ml wurde 1 ml für die OD₆₀₀-Messungₑₓₜₑᵣₙ genommen (entweder direkt in eine Küvette gegeben oder ins Eppi abgefüllt), der Rest wurde zur pH-Messungₑₓₜₑᵣₙ verwendet.

**Tabelle 2: Bei der Fermentation erhaltene Werte**

| **Zeit** | **Temp.** | **pO₂** | **pH intern 37°C** | **pH extern RT** | **OD intern** | **OD extern 600nm** | | **Luftzufuhr** | **Rührer** |
|---|---|---|---|---|---|---|---|---|---|
| | **°*C*** | ***%*** | | | ***CU**** | ***Verd.*** | | *l*/***min*** | ***rpm*** |
| 9:05 vorher | 34,6 | 96,1 | 6,45 recal. | 6,71 | 0,020 | x | X | < 1 | 396 |
| | | | **6,71** | | | | | | |
| 09:50 **vor Injekt.** | 37 | 101,1 | 6,71 | x | 0,022 | x | X | < 1 | 396 |
| 10:00 | 37 | 95,1 | 6,69 | 6,71 | 0,045 | - | 0,066 | < 1 | 396 |
| **Start** | | | | | | | 0,060 | | |
| 11:00 | 37 | 78,8 | 6,62 | 6,65 | 0,120 | - | 0,211 | < 1 | 396 |
| | | | | | | | 0,212 | | |
| 12:05 | 37 | 4,6 | 6,22 | 6,25 | 0,390 | 1+1 | 0,516 | < 1 | 396 |
| | | | | | | | 0,515 | | |
| 12:15 | 37 | 0,0 | 6,15 | x | 0,410 | x | X | < 1 | 396 |
| 12:50 | 37 | 20,1 | 5,91 | 5,99 | 0,520 | 1+4 | 0,282 | < 1 | 396 |
| | | | | | | | 0,278 | | |
| 13:50 | 37 | 91,1 | 5,98 | 6,04 | 0,330 | 1+1 | 0,283 | < 1 | 396 |
| | | | | | | | 0,279 | | |
| 14:50 | 37 | 99,0 | 6,05 | 6,08 | 0,220 | - | 0,316 | < 1 | 396 |
| | | | | | | | 0,314 | | |
| 15:50 | 37 | 100,4 | 6,08 | 6,12 | 0,190 | - | 0,230 | < 1 | 396 |
| | | | | | | | 0,230 | | |
| 16:50 | 37 | 101,0 | 6,10 | 6,14 | 0,190 | - | 0,189 | < 1 | 396 |
| | | | | | | | 0,184 | | |
| 17:15 | 37 | 101,3 | 6,11 | 6,14 | 0,180 | - | 0,179 | < 1 | 396 |
| | | | | | | | 0,176 | | |

| | | | | | | | | | |
|---|---|---|---|---|---|---|---|---|---|
| * aufgrund der Verwirbelungen bedingt durch das Rühren bei 400 rpm schwankten die Werte hin und her, so dass der hier dargestellte Wert lediglich eine Tendenz anzeigen kann X = keine Messung durchgeführt recal = Werte rekalibriert (Aufrufen über "Calibration" auf der Fermenteroberfläche, dann "Recalibration", Wert eingeben) | | | | | | | | | |

### 1.11 Ergebnisse

Es wurde eine Übernachtkultur verwendet, die nach OD-Anpassung auf 8,0 eine Konzentration von **8,5x10⁸ CFU/ml** (CFU = Kolonie bildende Einheit) aufwies.

### Berechnung MOI-Wert:

- Bakterien:: 15 ml x 8,5x10⁸ CFU = 1,3x10¹⁰ Bakterien pro ml
- Phagen:: 2 ml x 5x10⁷ PFU/ml (durch 2x10⁻²-Verdünnung) = 1x10⁸ PFU (Plaque bildende Einheiten pro ml)
- MOI:: 1/130 (Phagen/Bakterien) = 0,0077

Die Lyse setzte auch diesmal nach etwas weniger als 3 Stunden ein. Dieses scheint der zu erwartende Zeitraum für das Einsetzen der Lyse zu sein, denn bei einer Wiederholung der Fermentation war dieses zum selben Zeitpunkt der Fall. Der Fermentationsverlauf scheint sehr reproduzierbar zu sein, denn der Sauerstoffpartialdruck war nach jeweils 2 h 15 min auf 0,0 % gefallen und 30 min später wieder > 0,0 %. Auch die nach bestimmten Zeitabständen gemessene OD war über den Verlauf nahezu identisch. Die Bakterien erreichten dabei jeweils eine OD von 1,5 als ihren höchsten Wert. Als die Fermentation nach 7 h 15 min abgebrochen wurde, war noch eine OD₆₀₀ von 0,179 (A12: OD₆₀₀ 0,22) messbar. Allerdings zeigte die hohe Anzahl von lebensfähigen Staphylococcen auf dem Vogel-Johnson-Agar (s. Punkt 5.1.2) im Fermentationsprodukt an, dass die Fermentation/Lyse noch nicht zu 100 % abgeschlossen war.

Die in der Fermentation ermittelte Phagenkonzentration von 5,4x10⁹ PFU/ml liegt zwischen den Werten, die bei anderen Fermentationen erhalten wurden (3,3x10⁹ PFU/ml und 8,3x10⁹ PFU/ml). Eine Ausbeute von ca. 5x10⁹ PFU/ml wurde auch im Schüttelkolben erzielt, so dass dieses Ergebnis durchaus zufriedenstellend ist. Wenn die Lyse bei 4°C weiter fortschreitet, kann eine höhere Ausbeute erzielt werden.

### Beispiel 2 - Herstellung eines Bakteriophagengemisches

Zunächst wurde der Produktionsstamm, ein Methicillin-sensitiver Staphylococcus aureus-Stamm, der bei der DSM unter der Nummer 18421 hinterlegt wurde, über Nacht in etwa 250 ml Nährmedium bei einer Temperatur von 30°C kultiviert. Durch Verdünnung der angewachsenen Bakterienkulturbrühe mit frischem, sterilem Medium wurde eine optische Dichte von etwa 1,0 eingestellt. Die verdünnte Bakterienkultur wurde nun mit einer geeigneten Menge des Bakteriophagen so versetzt, dass ein Verhältnis von Phage zu Bakterium von etwa 1 eingestellt wurde. Anschließend wurde die Mischung von Bakterien und Bakteriophagen etwa 5 Stunden bei einer Temperatur von 30°C vorinkubiert.

Nach fünfstündiger Vorinkubation wurde das Bakterien/Bakteriophagengemisch in einen 10-Liter-Fermenter überführt, in dem der oben erwähnte Methicillin-sensitiver Staphylococcus aureus-Stamm bei 37°C auf eine optische Dichte von 2,0 angezogen wurde. Die vorgezogenen Bakterien, die mit der Vorkultur gemischt wurden, wurden für weitere 12 Stunden bei 37°C fermentiert. Danach wurde die Fermentation beendet und die Fermentationsbrühe enthaltend Bakterien, Bakterienreste und Phagen wurde zunächst in einer Tiefenfiltration vorgereinigt. Bei der Tiefenfiltration wird die Fermentationsbrühe durch ein Filterelement filtriert, das eine Porengröße aufweist, die die Passage der Bakteriophagen ohne Weiteres erlaubt. Da Bakteriophagen häufig negative Ladungen aufweisen, hat das Filterelement negative Ladungen, um eine unerwünschte Adsorption der Bakteriophagen an das Filterelement zu verhindern.

Nach der Tiefenfiltration wird die Fermentationsbrühe durch einen Filter mit einer Porengröße von etwa 0,1 bis 1 µm Durchmesser filtriert, um Bakterien sowie Bakterienbestandteile, insbesondere Membranbestandteile zu entfernen.

Da die nach der Mikrofiltration erhaltene Lösung enthaltend die Bakteriophagen sehr voluminös ist, wurde mit Hilfe einer Ultrafiltration das Volumen erheblich reduziert. Bei der Ultrafiltration ist die Porengröße des Filters so gewählt, dass nur das Lösungsmittel der Fermentationsbrühe, also Wasser sowie darin gelöste Bestandteile des Nährmediums durch das Filtern entfernt werden. Dadurch wird die Konzentration an Bakteriophagen erheblich erhöht, üblicherweise wird eine Konzentration der Bakteriophagen um einen Faktor zwischen 100 bis 10.000 erreicht.

Die konzentrierte Bakteriophagenmischung wurde anschließend mit Benzonase versetzt, wodurch die noch verbliebenen bakteriellen Nukleinsäuren entfernt werden. Anschließend wurde die Bakteriophagenlösung an einen Ionenaustauscher adsorbiert und anschließend mit einem Salzgraduenten eluiert. Die Fraktionen, die die Bakteriophagen enthalten, wurden anschließend über eine Gelchromatographie weiter gereinigt und die so erhaltene Bakteriophagenlösung wurde auf die gewünschte Konzentration der Bakteriophagen eingestellt. Für die Herstellung des Gemisches wurden die beiden erfindungsgemäßen bevorzugten Bakteriophagen separat angezogen und gereinigt und anschließend gemischt.

Die Bakteriophagenmischung wurde dann in eine gewünschte Form, also eine auftragbare Lösung, Salbe, Puder oder Granulat gebracht, abgefüllt und kann eingesetzt werden.

### Beispiel 3 - Stabilität der Bakteriophagen-Präparation bei 4°C, Raumtemperatur und bei 37°C

Es wurde als Lagerpuffer ein Phagenpuffer mit folgender Zusammensetzung eingesetzt: 20 mM Tris (pH 7,4), 100 mM NaCl, 10 mM MgSO₄x7H₂O. Die Herstellung erfolgte aus sterilen Stammlösungen sowie sterilem entionisiertem Wasser. Als Stabilisator wurde Human Serum Albumin (HSA) in einer Konzentration von 1 g/l eingesetzt. Das Produkt wurde von Baxter bezogen.

Untersucht wurden die beiden Bakteriophagen MP13.3 sowie KP3.

Für die Bestimmung der PFU wurde Staphylococcus aureus, hinterlegt bei der DSM unter der Nummer 18421 in LB-Medium angezogen. Mit der Übernachtkultur wurde ein 100 ml Erlenmeyerkolben mit 20 ml LB-Medium so angeimpft, dass eine OD₆₀₀ von 0,2 erreicht wird. Anschließend wird die Kultur bei 37°C und 200 rpm im Schüttelinkubator so lange inkubiert, bis eine OD₆₀₀ zwischen 0,8 und 1 erreicht wird.

Von der zu untersuchenden Phagenlösung wird eine dekadische Verdünnungsreihe in Phagenpuffer angelegt (100 µl Phagen + 900 µl Phagenpuffer).

Von den verdünnten Phagen werden jeweils 100 µl im Doppelansatz mit einer sterilen Filterspitze auf den Boden steriler, beschrifteter Zentrifugenröhrchen pipettiert. In ein Röhrchen werden 100 µl Phagenpuffer als Kontrolle gegeben. Anschließend werden alle Röhrchen mit 100 µl DSM 18421 (OD₆₀₀ 0,8 bis 1,0) versetzt. Die Röhrchen werden mit Alu-Deckeln verschlossen, vorsichtig geschüttelt und für 20 min bei 37 °C zusammen mit den zugehörigen Agarplatten im Brutschrank vorinkubiert.

Etwa 2 min vor Ablauf Vorinkubation wird LB-Topagar aus dem 60 °C-Schrank entnommen und 1-2 min bei Raumtemperatur auf einem Magnetrührer gemischt. Die angewärmten Platten, die Röhrchen und der Topagar werden nach der Inkubation unter der Cleanbench platziert.

6 ml des Topagar werden mit einer 5 ml Pipette (genaue Skalierung über 5 ml hinaus) aufgezogen und auf zwei Zentrifugenröhrchen zu je 3 ml verteilt. Die Röhrchen wurden für 2 Sekunden auf dem Vortexer gemischt und anschließend zügig auf den zugehörigen Agarplatten ausgegossen und verteilt. Die Agarplatten werden mindestens 10 min unter der Cleanbench getrocknet, bevor sie in den 37 °C-Brutschrank transportiert und "upside-down" über Nacht inkubiert werden.

**Tabelle 3**

| Stabilität von MP4 mit und ohne HSA | | | | | |
|---|---|---|---|---|---|
| Werte als PFU/ml | | | | | |
| **Tage** | **HSA** | **0** | **1** | **7** | **14** |
| **4°C** | ohne | 3,10E+09 | 3,00E+09 | 2,20E+09 | 1,70E+09 |
| | 1 mg/ml | 3,10E+09 | 2,70E+09 | 2,70E+09 | 2,30E+09 |
| **RT** | ohne | 3,10E+09 | 2,90E+09 | 2,10E+09 | 1,40E+09 |
| | 1 mg/ml | 3,10E+09 | 2,70E+09 | 2,70E+09 | 2,40E+09 |
| **37 °C** | ohne | 3,10E+09 | 1,30E+09 | 6,40E+07 | 1,20E+06 |
| | 1 mg/ml | 3,10E+09 | 2,30E+09 | 1,50E+09 | 6,50E+08 |

Die Tabelle 3 zeigt die Ergebnisse der Stabilisierung, die durch den Zusatz von Human Serum Albumin erzielt werden konnten am Beispiel des Bakteriophagen MP13.3. Die in Tabelle 3 verwendete Schreibweise ist eine andere Darstellung der Phagenkonzentration. So bedeutet beispielsweise 3,10 E + 09: 3,10 · 10⁹ PFU Phagen pro ml Lösung.

Es wurde auch die Langzeitstabilität am Beispiel von MP4 untersucht, wobei die Präparationen bei 4°C über eine Zeit von bis zu 32 Wochen gemessen wurde. Die Ergebnisse sind in der Tabelle 4 dargestellt. Die Abkürzung M-Phage (MP) steht für den Bakteriophagen MP13.3 (DSM 18722) und die Abkürzung K-Phage steht für den Bakteriophagen KP3 (DSM 18723).

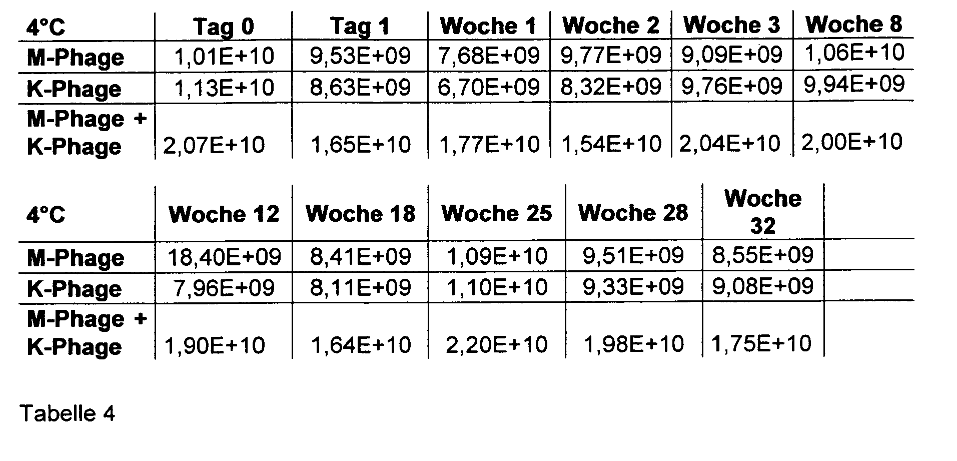

### Beispiel 4 - Bestimmung der spezifischen Aktivität

### a) Bestimmung der Proteinkonzentration

Coomassie Blue G-250 bildet im sauren Milieu mit Protein einen blauen Farbkomplex, der bei λ = 595 nm mit Hilfe eines Mikrotiter-Readers (96 Wells/Mikrotiterplatte, 300 µl/Well) photometrisch quantifiziert werden kann.

### Materialien:

Coomassie® Protein Assay Reagent (Fertiglösung) von Pierce (Bestellnummer: 23200) Standards (500 µl-Aliquots zu 1 mg/ml):
○ γ-Globulin (z.B. BioRad, Best.-Nr.: 500-0005);
○ Rinderserumalbumin (BSA) (z.B. BioRad, Best.-Nr.: 500-0007);

### Vorgehensweise:

Ein Aliqot der adäquat verdünnten Probe wurde zum Reagenz gegeben und innerhalb eines definierten Zeitintervall photometrisch vermessen. Aus der Standardkurve wurde unter Berücksichtigung des Verdünnungsfaktors die Gesamtproteinkonzentration in mg/l oder µg/l berechnet.

### b) Phagenaktivität

### Test-Prinzip:

Die quantitative Bestimmung der Infektiosität/Aktivität einer Phagensuspension erfolgt durch den Plaque-Test. Das Prinzip beruht auf der für Staphylococcus aureus spezifischen lytischen Wirkung des verwendeten Phagen. Durch die Vermehrung der Phagen innerhalb eines Wirtszellrasens entstehen klare, makroskopisch sichtbare Herde, sogenannte "Plaques", die deutlich von der Umgebung abgesetzt sind.

Genau ein Plaque kann, eine geeignete MOI (multiplicity of infection) vorausgesetzt, auf die Infektion einer Bakterienzelle mit einem einzelnen infektiösen Phagen zurückgeführt werden.

Anhand des eingesetzten Probenvolumens, der Probenverdünnung und der ermittelten Plaqueanzahl pro Platte kann die Phagenaktivität in PFU/ml (Plaque Forming Unit) oder PBE/ml (Plaque Bildende Einheit) berechnet werden.

Sofern der Phagentiter der zu vermessenden Probe bekannt ist, werden die Verdünnung und das Probevolumen so gewählt, dass die Anzahl der Plaques pro Agarplatte zwischen 50 und 250 liegt. Wenn der Phagentiter der Probe nicht genau bekannt ist, muss, über das Probevolumen oder die Verdünnungsstufen, ein breiteres Spektrum abgedeckt werden.

### Materialien:

Staphylococcus aureus DSM 18421.

### Vorgehensweise:

Anzucht des Staphylococcus aureus in einer Schüttelkultur in zwei Stufen.
Kurzzeitige Inkubation des Probenaliquots mit einem Aliquot der Staphylococcus-Kultur.
Mischen der Suspension mit flüssigem Agar und Ausplattierung des Agars auf einer vorbereiteten Agartestplatte.
Inkubation der Agartestplatte für 18 - 22 h.
Auszählung der Plaques.
Berechnung der Aktivität in PFU / ml.

### c) Spezifische Aktivität

Als spezifische Aktivität wird das Verhältnis der Aktivität zum Protein in PFU/µg Protein definiert. Es wurden folgende Werte gemessen:

**Tabelle 5**

| | **Gesamtprotein (**µ**g/ml)** | **Aktivität (PFU/ml)** | **Spezifische Aktivität (PFU/**µ**g Protein)** |
|---|---|---|---|
| **K-Phage** | 25,5 | 2,42E+10 | 0,09E+10 |
| **M-Phage** | 26,5 | 6,48E+10 | 0,24E+10 |

Die erfindungsgemäß eingesetzten Phagen weisen in der eingesetzten Lösung bevorzugt eine spezifische Aktivität von wenigstens 10⁸ PFU/µg Protein und besonders bevorzugt eine spezifische Aktivität von 9^{·}10⁸ (entspricht 0,09E+10) für den K-Phagen und 2,4^{·}10⁹ (0,24E+10) für den M-Phagen auf (jeweils PFU/µg Protein). Bei verschiedenen Chargen wurden die folgenden Werte ermittelt:

**Tabelle 6**

| Ch.-Bez. | Gesamtprotein (µg/ml) | Aktivität (PFU/ml) | Spez. Aktivität (PFU/µg Protein |
|---|---|---|---|
| SA1-ABT-031 (M-Phage) | 27 | 6,48E+10 | 0,24E+10 |
| SA1-ABT-032 (M-Phage) | 26 | 5,07E+10 | 0,20E+10 |
| SA1-ABT-034 (K-Phage) | 26 | 5,19E+10 | 0,20E+10 |
| SA1-ABT-035 (K-Phage) | 27 | 2,42E+10 | 0,09E+10 |

### Beispiel 5 - Einsatz von Benzonase

Benzonase ist eine Endonuklease, die DNA sowie auch RNA hocheffizient zu kleinsten Einheiten abbaut. Dieses Enzym wird vor allem zur Verminderung der Viskosität eingesetzt.

Ein weiteres Einsatzgebiet ist vor allem die Verhinderung der Interaktion zwischen DNA und großen Proteinen. Diese Interaktion kann eine effiziente Aufreinigung erschweren oder gar verhindern. Dies gilt insbesondere für den vorliegenden Prozess, bei dem die sehr große Proteinhülle des Phagen zu berücksichtigen ist. Das erfindungsgemäße Verfahren wurde einmal ohne Benzonase- und einmal mit Benzonase-Behandlung durchgeführt. Die Ergebnisse sind in Tabelle 7 und 8 wiedergegeben.

**Tabelle 7: Ionenaustauscherchromatographie ohne Benzonase**

| Fraktion | Bakteriophagen PFU total | Wiederfindung % | O.D. 260/280 | Protein µg | Spez. Aktivität PFU/µg |
|---|---|---|---|---|---|
| *Ausgang* | *16***10¹⁰* | | *1,72* | 545,7 | *0,029***10¹⁰* |
| Peak 1 | 4,2*10¹⁰ | 26 | 1,28 | 31 | 0,135*10¹⁰ |
| Peak 2 | 0,9*10¹⁰ | 6 | 1,79 | 16 | 0,056*10¹⁰ |
| | 17,6 % Verlust | 32 | | | |

**Tabelle 8: Ionenaustauscherchromatographie mit Benzonase**

| Fraktion | Bakteriophagen PFU total | Wiederfindung % | O.D. 260/280 | Protein µg | Spez. Aktivität PFU/µg |
|---|---|---|---|---|---|
| *Ausgang* | *114***10¹⁰* | | *1,72* | *785,7* | *0,145***10¹⁰* |
| Peak 1 | 49,1*10¹⁰ | 43 | 1,33 | 199,5 | 0,25*10¹⁰ |
| Peak 2 | 0,6*10¹⁰ | 1 | 1,96 | 60 | 0,01*10¹⁰ |
| | 1,2 % Verlust | 44 | | | |

### Beispiel 6 - Wirksamkeit des Gemisches

Um zu belegen, dass die erfindungsgemäße Mischung von Bakteriophagen überraschende Eigenschaften aufweist, wurden verschiedene Problemerreger aus einer Klinik getestet. Bei den eingesetzten Staphylococcus aureus-Stämmen mit multipler Antibiotika-Resistenz handelte es sich um Isolate, die von einer Klinik zur Verfügung gestellt wurden.

Es wurde überprüft, ob die verschiedene Staphylococcus aureus-Isolate einmal durch den MP13.4-Phagen, einmal durch den Phagen KP4 und einmal durch ein 1:1-Gemisch von MP13.4 und KP4 lysiert werden konnten.

Die nachfolgende Tabelle 9 zeigt, dass durch den Einsatz eines Phagengemisches auch solche Staphylococcus aureus-Stämme lysiert werden können, die durch einen einzigen Bakteriophagen nicht lysiert werden können. Da in der Klinik bei der Therapie von multiplen Antibiotika-resistenten Staphylococcen-Stämmen diese Erreger möglichst schnell und wirkungsvoll lysiert werden müssen, ist es von unschätzbarem Vorteil, wenn ein Bakteriophagen-Gemisch möglichst viele unterschiedliche Problemkeime lysieren und damit vernichten kann.

**Tabelle 9: Vergleich der Wirksamkeit der Bakteriophagen sowohl einzeln wie auch als Gemisch bei resistenten Staphylococcus aureus-Stämmen**

| **Nr.** | **Stamm*** | **MP13.4** 1x10⁸ (PFU/ml) | **KP4** 1x10⁸ (PFU/ml) | **Mix MP13.4 + KP4 1:1** 1x10⁸ (PFU/ml) |
|---|---|---|---|---|
| 1 | MRSA 0151 | 0 | +++ | +++ |
| 2 | MRSA 0166 | +++ | +++ | +++ |
| 3 | MRSA 0226 | +(+) | + | +(+) |
| 4 | MRSA 0232 | +++ | (+) | +++ |
| 5 | MRSA 0486 | +++ | +++ | +++ |
| 6 | MRSA 0703 | +++ | (+) | +++ |
| 7 | MRSA 0975 | 0 | +++ | +++ |
| 8 | MRSA 1188 | +++ | (+) | +++ |
| 9 | MRSA 0811 | +++ | +++ | +++ |
| 10 | MRSA 1204 | +++ | + | +++ |
| 11 | S.a. 12535-2 | +++ | 0 | +++ |

| | | | | |
|---|---|---|---|---|
| * Bei den Stämmen handelt es sich um problematische Isolate aus einer Klinik, die gegenüber einer Vielzahl von Antibiotika resistent sind. 0 bedeutet keine Lyse; + bedeutet schwache Lyse, ++ bedeutet bessere Lyse und +++ bedeutet sehr gute Lyse | | | | |

### Beispiel 7 - Lyse von Staphylococcus aureus DSM 18421 in Speichel

### 7.1 Herstellung einer Speichel-Mischung

- von verschiedenen Personen wird Morgenspeichel (vor dem Frühstück und vor dem Zähneputzen) einzeln gesammelt
- die Speichelproben werden für 5 min bei Raumtemperatur mit 12.000 rpm zentrifugiert
- der Überstand wird abgenommen und steril filtriert (0,8 µm-Vorfilter, dann 0,22 µm)
- die Speichelproben werden 1:1 in einem sterilen Gefäß gemischt.

### 7.2 Inkubation der Lyseansätze

- in den wie oben beschriebenen präparierten Speichel werden weiterhin gegeben: ca. 1x10⁴ CFU/ml DSM 18421 (aus geeigneter Verdünnung der Übernachtkultur) ca. 1x10⁸ PFU/ml Phagen (Simba Top, 1:1-Mischung von aufgereinigten M- und K-Phagen)
- zusätzlich werden Serralysin (100 U/ml), Dornase (25 U/ml) oder Benzonase (25 U/ml) bzw. eine Kombination der Enzyme verwendet
- die Enzyme werden gleich zum Start der Inkubation zugesetzt
- es wird eine Wachstumskontrolle mitgeführt, bei der die Phagen durch Zugabe desselben Volumens von Lagerpuffern (Zusammensetzung s.u.) ersetzt werden
- alle Ansätze enthalten in der Summe 2 ml
- die Inkubation wird in 10 ml Penicillin-Glasflaschen bei 37°C und 200 rpm (Inkubationsschüttler) durchgeführt.

### 7.3 Bestimmung der Lebendzellzahl (CFU/ml) nach 6 h

- nach 6 h Inkubationsdauer wird aus jedem Ansatz ein Aliquot entnommen und dekadisch in Lagerpuffer verdünnt
- aus jeder Verdünnungsreihe werden 2-5 geeignete Verdünnungsstufen ausgewählt und jeweils 2x100 µl (Doppelansatz) davon ausplattiert (mittels Drigalski-Spatel)
- für die Ansätze ohne Phagen wird auf LB-Agar, für Ansätze mit Phagen auf LB-Agar mit 0,01 % Citrat ausplattiert
- nach mind. 16 h Inkubation bei 37°C werden die Kolonien (Colony forming units; CFU) auf jeder Platte ausgezählt
- für die Berechnung der CFU/ml werden nur Platten verwendet, die mind. 20 CFU haben
- die graphische Darstellung erfolgt durch die Auftragung der CFU/ml (logarithmisiert) über die Inkubationsdauer.

Die Ergebnisse des Versuches sind in Tabellen 10 bis 12 zusammengefasst.

**Tabelle 10: Serralysin mit und ohne Phagen:**

| **Ansatz** | | **CFU/mL** | **%** |
|---|---|---|---|
| Kontrolle ohne Phagen | | 8,80 x 10⁵ | 100 |
| Kontrolle ohne Phagen | | 1,14 x 10⁶ | 130 |
| | + Serralysin | | |
| | + Phagen | 5,93 x 10⁵ | 67,4 |
| | + Phagen + Serralysin | 1,65 x 10⁵ | 18,7 |

Tabelle 10 (Serralysin mit und ohne Bakteriophagen) beschreibt an einem Beispiel den Einfluss von Serralysin auf die Konzentration von S.aureus in Speichel allein und in Kombination mit Bakteriophagen (Gemisch von DSM 18722 und DSM 18723). Wie die Tabelle zeigt, hat Serralysin allein keine Reduktion der Kolonienzahl zur Folge. Das Bakteriophagengemisch zeigt eine Reduktion der Kolonienzahl auf 67,4 Prozent. In der Kombination von Bakteriophagen und Serralysin sinkt die Kolonienzahl auf 18,7 Prozent. Somit ist ein synergistischer Effekt gegeben.

**Tabelle 11: Vergleich Dornase α / Benzonase**

| **Ansatz** | **CFU/mL** | **%** |
|---|---|---|
| Kontrolle ohne Phagen | 3,31 x 10⁵ | 100 |
| Kontrolle + Phagen | 6,20 x 10⁴ | 18,7 |
| + Phagen + Dornase α | 2,94 x 10⁴ | 8,88 |
| + Phagen + Benzonase | 2,11 x 10⁴ | 6,37 |

Tabelle 11 (Vergleich Dornase α / Benzonase) beschreibt an einem Beispiel den Einfluss der Nukleasen Dornase α und Benzonase auf die Konzentration von S.aureus im Speichel. Der Einsatz von Bakteriophagen führt zu einer Reduktion der Kolonienzahl auf 18,7 Prozent. Die Kombination von Bakteriophagen und Nuklease reduziert die Kolonienzahl auf 8,88 Prozent bei Dornase α und 6,37 Prozent bei Benzonase.

**Tabelle 12: Vergleich Kombination Dornase α / Serralysin mit Einzel-Enzymen**

| **Ansatz** | **CFU/mL** | **%** |
|---|---|---|
| Kontrolle ohne Phagen | 4,97 x 10⁶ | 100 |
| Kontrolle + Phagen + Dornase α | 2,70 x 10⁶ | 54,3 |
| + Phagen + Serralysin | 1,40 x 10⁶ | 28,2 |
| + Phagen + Dornase + Serralysin | 9,95 x 10⁵ | 20,0 |

| | | |
|---|---|---|
| Inkubationsdauer: 6 Std. Medium: Speichel (human; Mischung) | | |

Tabelle 12 (Vergleich Kombination Dornase α / Serralysin mit Einzel-Enzymen) beschreibt an einem Beispiel den Vergleich der Wirkung der Nuklease Dornase α und der Proteinase Serralysin einzeln und in Kombination auf den Synergieeffekt mit Bakteriophagen. Die Enzyme reduzieren alleine die Kolonienzahl von S.aureus im Speichel auf 54,3 Prozent (Dornase α) und auf 28,2 Prozent (Serralysin). Die Kombination von beiden Enzymen führt zu einer weiteren Reduktion der Kolonienzahl auf 20,0 Prozent.

## Patentansprüche

1. Verfahren zur Herstellung eines Gemisches von Bakteriophagen, bei dem wenigstens zwei unterschiedliche Bakteriophagenstämme separat vermehrt wurden, wobei ein Staphylococcus-Stamm mit je einem Stamm eines Bakteriophagen, der Staphylococcen lysieren kann, in einer Vorkultur angezogen und dann in einer Hauptkultur weiter vermehrt und anschließend gereinigt wird und die verschiedenen Stämme anschließend gemischt werden, **dadurch gekennzeichnet, dass** das eingesetzte Gemisch von Bakteriophagen wenigstens zwei verschiedene Serotypen von Bakteriophagen enthält, die spezifisch Bakterien der Spezies Staphylococcus lysieren.

2. Verfahren nach Anspruch 1, **dadurch gekennzeichnet, dass** der zur Vermehrung der Bakteriophagen verwendete Bakterienstamm ein Methicillin-sensitiver Staphylococcus aureus-Stamm ist.

3. Verfahren nach Anspruch 2, **dadurch gekennzeichnet, dass** der Methicillin-sensitive Staphylococcus aureus-Stamm die Hinterlegungsnummer DSM 18421 hat.

4. Verfahren nach einem der vorhergehenden Ansprüche, **dadurch gekennzeichnet, dass** das in Vorkultur angezogene Gemisch von Bakterien und Bakteriophagen zu einer vorgezogenen Kultur von Staphylococcen gegeben wird, wobei das Verhältnis von Phagen zu Bakterien so eingestellt wird, dass der MOI-Wert zwischen 0,01 und 1 liegt.

5. Verfahren nach einem der vorhergehenden Ansprüche, **dadurch gekennzeichnet, dass** die Reinigung der Bakteriophagen eine lonenaustausch-Chromatographie und/oder eine Benzonasebehandlung umfasst.

6. Verfahren nach einem der vorhergehenden Ansprüche, **dadurch gekennzeichnet, dass** das Bakteriophagen-Gemisch den Bakteriophagen MP13.3 mit der Hinterlegungsnummer DSM 18722 und den Bakteriophagen KP3 mit der Hinterlegungsnummer DSM 18723 enthält.

7. Phagengemisch, **dadurch gekennzeichnet, dass** die Phagen eine spezifische Aktivität von wenigstens 10⁸ plaque-forming units pro µg Protein aufweisen.

8. Phagengemisch nach Anspruch 7, **dadurch gekennzeichnet, dass** das Gemisch den Bakteriophagen MP 13.3 (DSM 18722) und/oder den Bakteriophagen KP3 (DSM 18723) mit einer spezifischen Aktivität von jeweils wenigstens 10⁸ PFU/µg Protein enthält.

9. Phagengemisch nach einem der Ansprüche 7 oder 8, **dadurch gekennzeichnet, dass** es Humanserumalbumin als Stabilisator umfasst.

10. Phagengemisch nach einem der Ansprüche 7 bis 9, erhältlich nach einem Verfahren gemäß einem der Ansprüche 1 bis 6.

11. Arzneimittel, **dadurch gekennzeichnet, dass** es ein Phagengemisch nach einem der Ansprüche 7 bis 10 beinhaltet.

12. Arzneimittel nach Anspruch 11, **dadurch gekennzeichnet, dass** es zusätzlich ein gegen gram-positive Bakterien wirksames Antibiotikum enthält.

13. Arzneimittel nach Anspruch 11 oder 12, **dadurch gekennzeichnet, dass** es weiterhin ein Enzym enthält.

14. Arzneimittel nach Anspruch 13, **dadurch gekennzeichnet, dass** das Enzym ausgewählt ist aus der Gruppe bestehend aus Lysinen, Proteasen, Peptidasen, Nukleasen und/oder Glucosaminidasen.

15. Verwendung eines Phagengemisches nach einem der Ansprüche 7 bis 10 zur Therapie von Infektionen, die durch Antibiotika-resistente Staphylococcen verursacht werden.

16. Verwendung nach Anspruch 15, **dadurch gekennzeichnet, dass** es sich bei den Infektionen um Infektionen der Haut und/oder Schleimhäute handelt.

17. Verwendung nach Anspruch 15, **dadurch gekennzeichnet, dass** die Infektionen durch gegen mehrere Antibiotika resistente Staphylococcen verursacht werden.
